**Europäisches Patentamt**

**European Patent Office**

**Office européen des brevets**

(19)

(11) Veröffentlichungsnummer: **0 275 519 B1**

## EUROPÄISCHE PATENTSCHRIFT

(12)

(45) Veröffentlichungstag der Patentschrift: **13.03.91**

(51) Int. Cl.⁵: **C07C 255/52, C07C 253/00**

(21) Anmeldenummer: **87118898.3**

(22) Anmeldetag: **19.12.87**

(54) Verfahren zur Herstellung von 1,4-Diamino-2,3-dicyanoanthrachinon.

(30) Priorität: **31.12.86 DE 3644824**

(43) Veröffentlichungstag der Anmeldung:
**27.07.88 Patentblatt 88/30**

(45) Bekanntmachung des Hinweises auf die
Patenterteilung:
**13.03.91 Patentblatt 91/11**

(84) Benannte Vertragsstaaten:
**BE CH DE FR GB IT LI NL**

(56) Entgegenhaltungen:
**EP-A- 0 023 645**
**EP-A- 0 179 740**
**FR-A- 1 475 602**
**FR-A- 2 468 585**
**GB-A- 2 183 668**

(73) Patentinhaber: **BASF Aktiengesellschaft**
**Carl-Bosch-Strasse 38**
**W-6700 Ludwigshafen(DE)**

(72) Erfinder: **Miederer, Peter, Dr.**
**Barbarossastrasse 3**
**W-6733 Hassloch(DE)**
Erfinder: **Michaelis,Eberhard, Dr.**
**Schlossgasse 8**
**W-6721 Weingarten(DE)**

EP 0 275 519 B1

## Beschreibung

1,4-Diamino-2,3-dicyanoanthrachinon - im folgenden auch als Diaminodinitril bezeichnet - ist ein wichtiges Ausgangsmaterial zur Herstellung blauer anthrachinoider Farbstoffe. Zur Herstellung des Dinitrils sind verschiedene Wege bekannt.

So wird in der DE-A-3 039 262 die Umsetzung von 1,4-Diamino-anthrachinon-2-sulfonsäure, im folgenden Diaminosäure genannt, oder deren Salz, von 1,4-Diamino-2-cyananthrachinon oder von 1,4-Diaminoanthrachinon-2,3-disulfonsäure oder deren Salz in einem wäßrigen Medium mit einer Cyanidionen abgebenden Verbindung in Gegenwart quartärer Ammoniumverbindungen beschrieben.

Nach den Angaben in den Beispielen 1 und 3 werden - bezogen auf Diaminosäure - die 3,3fache Gewichtsmenge Benzyltriethylammoniumchlorid, bzw. die 5,5fache Menge Lauryltrimethylammoniumchlorid angewendet. Die Aufarbeitung derartiger Mengen an organischen Ballaststoffen enthaltenden Reaktionsgemischen ist problematisch. Die Entsorgung über das Abwasser stellt eine hohe Belastung an organischem Kohlenstoff dar und ist auch aus wirtschaftlichen Gründen nicht zu vertreten. Auch die zusätzliche Behandlung des Abwassers durch die angegebene Extraktion mit einem organischen Lösungsmittel ist keine wirtschaftliche Alternative.

In der DE-A-2 931 981 wird die Umsetzung von 1,4-Diaminoanthrachinon-2-sulfonsäure oder 1,4-Diaminoanthrachinon-2,3-disulfonsäure mit Cyanidionen abgebenden Verbindungen in Formamid zu Diaminodinitril beschrieben. Abgesehen davon, daß bei der Nacharbeitung die angegebenen Ausbeuten nicht erreicht werden können, sind bei diesem Verfahren hohe Lösungsmittel- und Abwasserkosten oder bei einer Regeneration des cyanidhaltigen Lösungsmittels ein beträchtlicher technischer Aufwand, verbunden mit entsprechenden Kosten, zu erwarten.

Wäßrige Verfahren zur Herstellung von Diaminodinitril aus Diaminosäure durch Umsetzen mit Salzen der Cyanwasserstoffsäure in Abwesenheit oder Anwesenheit von Oxidationsmitteln sind in den DE-C-536 998 und 1 108 704 beschrieben. Wegen der großen Verdünnung - das Verhältnis Diaminosäure zu Wasser beträgt 1:50 - sind die nach diesem Verfahren erzielbaren Raum-Zeit-Ausbeuten recht gering. Das Verfahren hat außerdem den Nachteil, daß das Verfahrensprodukt über 10 Gew.% an störenden Nebenprodukten wie 1,4-Diaminoanthrachinon, 1,4-Diamino-2-cyanoanthrachinon (Mononitril) und 1,4-Diaminoanthrachinon-2,3-dicarbonsäureimid enthält, was zu Lasten der Ausbeute an gewünschtem Produkt geht. Nach den Angaben in den Beispielen 1 bis 4 der DE-C-1 108 704 sind hohe Überschüsse an Natriumcyanid bezogen auf die Diaminosäure erforderlich (Diaminosäure:Natriumcyanid = 1:7,5 bis 1:9,5 Mol). Hierdurch entstehen erhöhte Kosten für Material und für die Vernichtung des Überschusses an Cyanid.

Aufgabe der Erfindung war es, ein Verfahren zur Herstellung von Diaminodinitril aus Diaminosäure bereitzustellen, bei dem keine kostspielige Abwasseraufbereitung nötig ist und das bei guter Raum-Zeit-Ausbeute eine bessere Produktqualität liefert als die Verfahren des Standes der Technik.

Erfindungsgemäß wird diese Aufgabe dadurch gelöst, daß die Umsetzung mit Natriumcyanid zum Dinitril in einem speziellen wäßrigen Reaktionsmedium erfolgt.

Die Erfindung betrifft dementsprechend ein Verfahren zur Herstellung von 1,4-Diamino-2,3-dicyanoanthrachinon durch Umsetzen von 1,4-Diaminoanthrachinon-2-sulfonsäure, bzw. von deren Salz, mit Cyanidionen in Wasser im pH-Bereich von 8 bis 11 in Gegenwart von oxidierend wirkenden Mitteln in der Wärme, das dadurch gekennzeichnet ist, daß die Umsetzung in einem Gemisch aus Wasser und 1-Methoxipropanol-2, 2-Methoxipropanol-1, N,N-Dimethylformamid, N,N-Dimethylacetamid, N,N-Dimethylpropionsäureamid, Dimethylsulfoxid, Dioxan, N-Methylpyrrolidon oder Gemischen davon erfolgt, wobei das Verhältnis Wasser zu organischer Flüssigkeit ≧ 3:1 Gew.-Teile ist und wobei das fieuish auf den fur deu zu Begiuu der Uuisefzuug erforderlicheu ph-Bereid vou 8-g gepuffert wird.

Dieser gunstige Effekt des erfindungsgemaßen Reaktionsmediums auf die Reaktion war nicht vorhersehbar. Verwendet man anstelle der genannten organischen Flüssigkeiten z.8. andere Alkohole wie Ethylenglykol, Ethylenglykolmonomethylether-Oiethylenglykolmonomethylether, Ethylenglykolmonobutylether, Diethylenglykolmonobutylether, Ethylenglykolmonophenylether, n-Propanol, Isobutanol- n-Butanol, n-Pentanol, Dipropylenglykolmonomethylether oder Dipropylenglykolglykolmonoisopropylether dauert die Reaktion in der Regel langer und die Ausbeuten sind deutlich niedriger als 2.8. bei der Verwendung von einem 1-Hethoxipropanol-2-/Uasser-Gomisch.

Das erfindungsgemaße Gemisch aus den organischen Flussigkeiten und Hasser ermoglicht die Cvanierungsreaktion bei hoheren Konzentrationen als in rein waßrigem Medium durchzufuhren. Dadurch wird die Selektivitat der Reaktion erhöht und das Endprodukt Diaminodinitril entsteht mit höherer Reinheit bei gleichzeitig hoher Raum-Zeit-Ausbeute. iseiterhin wirkt sich der Zusatz der erfindungsgemäßen organischen Flüssigkeiten positiv auf die Reaktionszeit aus. Im Gegensatz zur Umsetzung im rein wäßrigen Medium oder in Gemischen aus Wasser und anderen Lösungsmitteln erfolgt die Reaktion bei dem erfindungsgemäßen Verfahren bedeutend rascher, was sich zusätzlich günstig auf die Raum-Zeit-Ausbeute auswirkt.

Im allgemeinen führt man das erfindungsgemäße Verfahren so durch: die Diaminosäure wird zu dem auf 60 bis 80° C erwärmten Reaktionsmedium und gegebenenfalls Natronlauge gegeben und die entstandene Suspension oder Lösung auf pH = 7 bis 8 gestellt. Danach wird auf pH = 8 bis 9 gepuffert und Oxidationsmittel zugegeben. Nach dem Zufügen der Cyanidionen abgebenden Verbindung erfolgt bei 60 bis 95° C die Reaktion mit den Cyanidionen zum Diaminodinitril. Das Ende der Umsetzung ist dünnschichtchromatografisch leicht erkennbar. Nach Vernichtung des Cyanidüberschusses, beispielsweise mit Wasserstoffperoxid, wird das Reaktionsgemisch filtriert, der Rückstand mit Wasser gewaschen und getrocknet. Als Reaktionsmedium dient erfindungsgemäß ein Gemisch aus Wasser und 1-Methoxipropanol-2, 2-Methoxypropanol-1, Dimethylformamid, N-Methylpyrrolidon, Dimethylsulfoxid, Dimethylacetamid, Dimethylpropionsäureamid oder Dioxan oder ein Gemisch dieser Flüssigkeiten mit Wasser. Als organische Flüssigkeit sind 1-Methoxipropanol-2, Dimethylformamid, Dimethylacetamid, N-Methylpyrrolidon, Dimethylsulfoxid und Dioxan bevorzugt. Das Verhältnis Wasser zu organischer Flüssigkeit ist ó>ò 3:1, vorzugsweise 10:1 bis 4:1. Dementsprechend beträgt der Gehalt des Reaktionsmediums an der organischen Flüssigkeit etwa 7,5, vorzugsweise 9 bis 20 Gew.%, bezogen auf das Medium. Höhere Konzentrationen an der organischen Flüssigkeit sind nicht nachteilig, bringen jedoch auch keine Vorteile.

Die Menge des Reaktionsmediums beträgt in der Regel das 2,5- bis 5fache der Gewichtsmenge an Diaminosäure. Die Menge an Natronlauge wird zweckmäßigerweise so gewählt, daß die Sulfonsäure praktisch neutralisiert wird und in Lösung geht. Anschließend wird die Lösung durch Zugabe weiterer Natronlauge oder auch Säure auf einen pH-Wert zwischen 7 und 8 eingestellt. Danach wird das Gemisch auf den für den zu Beginn der Umsetzung erforderlichen pH-Bereich von 8 bis 9 gepuffert. Als Puffersubstanz hat sich Natriumhydrogencarbonat vorteilhaft erwiesen, das in einer Menge von 50 bis 100 Gew.%, vorzugsweise von 60 bis 80 Gew.%, bezogen auf eingesetzte Diaminosäure, zu der auf pH 7 bis 8 gestellten Lösung gegeben wird. Anstelle von Natriumbicarbonat können auch Hydrogenphosphate oder leicht verseifbare Ester wie Ethylenglykolacetat als Puffer verwendet werden. Die Umsetzung erfolgt im pH-Bereich 8 bis 11. Bei pH-Werten > 11 findet in vermehrtem Maße eine Hydrolyse der Cyangruppen im Diaminodinitril statt, wodurch die Ausbeute vermindert wird. Nach Ende der Reaktion liegt der pH-Wert zwischen 9 und 10.

Als Oxidationsmittel wird vorteilhafterweise m-Nitrcbenzolsulfonsäure/Natriumsalz verwendet. Man kann jedoch auch Luft verwenden, die während der Reaktion durch das Reaktionsgemisch geleitet wird. Da hierbei Cyanwasserstoff mit der Luft entweicht, der vernichtet werden muß, ist diese Variante weniger zu empfehlen.

Als Cyanidionen abgebende Verbindungen kommen vorzugsweise die Alkalimetallcyanide in Betracht. Da die Handhabung der festen Cyanide in größeren Mengen problematisch ist, verwendet man daher in der Regel 20 bis 30 gew.%ige wäßrige Lösungen. Das auf diese Weise in das Reaktionsgemisch eingebrachte Wasser muß bei der Bemessung des Anteils an der organischen Flüssigkeit berücksichtigt werden. Die Menge an Cyanid abgebender Verbindung beträgt 3 bis 5 Mol Natriumcyanid je Mol Diaminosäure.

Die Reaktion erfolgt bei 60 bis 100, vorzugsweise bei 90 bis 95° C und ist unter den angegebenen Bedingungen im allgemeinen nach 3 bis 7 Stunden beendet. Bei höheren Temperaturen findet eine vermehrte Hydrolyse der Cyangruppen am Diaminodinitril auf Kosten der Ausbeute statt.

Die folgenden Ausführungsbeispiele sollen die Erfindung zusätzlich erläutern. Die Teile und Prozentangaben beziehen sich auf das Gewicht.

Beispiel 1

Zu einem auf 80° C erwärmten Gemisch aus 160 Teilen Wasser und 20 Teilen 1-Methoxipropanol-2 werden 65,4 Teile 1,4-Diaminoanthrachinon-2-sulfonsäure (Diaminosäure; Reingehalt: 91,7 %) eingetragen. Die Suspension wird mit 16 Teilen 50 %iger Natronlauge auf einen pH-Wert von 7,5 bis 8,0 eingestellt, wozu 16 Teile der Lauge benötigt werden. Dabei tritt vollständige Lösung der Sulfonsäure ein. Nach Zugabe von 15,6 Teilen m-Nitrobenzolsulfonsäure (Natriumsalz), 48 Teilen Natriumhydrogencarbonat und 37,2 Teilen Natriumcyanid wird das Gemisch auf 90° C geheizt und 7 Stunden bei dieser Temperatur gerührt. Nach dem Dünnschichtchromatogramm ist die eingesetzte Sulfonsäure dann vollständig umgesetzt.

Durch portionsweise Zugabe von 35 Teilen 30 %igem Wasserstoffperoxid in die auf 60° C abgekühlte Suspension wird überschüssiges Natriumcyanid vernichtet. Die heiße Suspension wird abgesaugt, der Rückstand wird mit heißem Wasser gewaschen und bei 100° C getrocknet.

Ausbeute: 53,9 Teile Diaminodinitril mit einem Reingehalt von 81,5 % und einem Gehalt von 2,4 % an Mononitril. Die Ausbeute entspricht 80,8 % d.Th.

Beispiel 2

Eine auf 80°C erwärmte Mischung aus 84 Teilen Wasser und 20 Teilen 1-Methoxipropanol-2 wird mit 65,4 Teile Diaminosäure (Reingehalt: 91,7 %) versetzt und mit ca. 16 Teilen 50 %iger Natronlauge auf einen pH-Wert zwischen 7,5 und 8,0 eingestellt. Danach werden 15,6 Teile m-Nitrobenzolsulfonsäure/Natriumsalz, 48 Teile Natriumhydrogencarbonat und 124 Teile einer 30 %igen wäßrigen Natriumcyanidlösung zugesetzt. Nach 5stündigem Rühren bei 90°C ist die Reaktion beendet und der Ansatz wird wie in Beispiel 1 aufgearbeitet.

Ausbeute: 53,2 Teile Diaminodinitril mit einem Reingehalt von 82,3 %; die Ausbeute beträgt 80,6 % d.Th., bezogen auf die eingesetzte Sulfonsäure. Gehalt an Mononitril: 2,8 %

Beispiel 3

In eine 60°C heiße Mischung aus 738 Teilen 1-Methoxipropanol-2, 600 Teilen 50 %ige Natronlauge und 2960 Teilen Wasser werden 2616 Teile 1,4-Diaminoanthrachinon-2-sulfonsäure (Reinheit: 91,7 %) eingetragen. Das Gemisch wird mit 50 %iger Natronlauge auf pH 7,5 und 8,0 eingestellt, wozu 60 Teile erforderlich waren. Anschließend werden 650 Teile m-Nitrobenzolsulfonsäure (Natriumsalz), 1520 Teile Natriumhydrogencarbonat und zuletzt 4960 Teile einer 30 %igen wäßrigen Natriumcyanidlösung zugegeben und das Reaktionsgemisch auf 90°C erwärmt. Nach 5stündigem Rühren bei 90°C ist die Sulfonsäure vollständig umgesetzt. Der pH-Wert ist 9,5. Der Reaktionsansatz wird wie in Beispiel 1 aufgearbeitet.

Ausbeute: 2180 Teile 1,4-Diaminoanthrachinon-2,3-dicarbonsäurenitril mit einem Reingehalt von 81,1 %. Die Ausbeute entspricht 81,3 % d.Th., bezogen auf die reine Sulfonsäure. Der Gehalt an Mononitril beträgt 2,1 %.

Beispiele 4 bis 8

Man verfährt wie in Beispiel 1, verwendet jedoch anstelle von 20 Teilen 1-Methoxipropanol-2 jeweils 20 Teile der in der Tabelle, Spalte 2, angegebenen Lösungsmittel. Ausbeute und Reingehalt des erhaltenen Diaminodinitrils sowie der Gehalt an Mononitril sind in der folgenden Tabelle zusammengestellt.

| | Lösungsmittel | Diaminodinitril | | Gehalt an |
| | | Ausbeute | Reingehalt | Mononitril |
| | | % d.Th. | % | % |
| --- | --- | --- | --- | --- |
| Beispiel 4 | Dimethylacetamid | 80,4 | 83,1 | 3,2 |
| Beispiel 5 | N-Methylpyrrolidon | 81,8 | 84,0 | 2,2 |
| Beispiel 6 | Dimethylformamid | 80,4 | 83,1 | 3,7 |
| Beispiel 7 | Dioxan | 76,3 | 81,3 | 2,7 |
| Beispiel 8 | Dimethylsulfoxid | 79,4 | 80,0 | 3,2 |

Vergleichsbeispiel 1

Eine Suspension von 65,4 Teilen 1,4-Diaminoanthrachinon-2-sulfonsäure (Reingehalt: 91,7 %) in 180 Teilen Wasser wird mit 50 %iger Natronlauge auf einen pH-Wert von 7,5 und 8,0 gestellt, erforderlich waren 16 Teile. Zu der entstandenen Lösung werden anschließend 15,6 Teile m-Nitrobenzolsulfonsäure/Natriumsalz, 48 Teile Natriumhydrogencarbonat, 0,4 Teile Ammoniumvanadat und 37,2 Teile Natriumcyanid gegeben. Nach 20stündigem Rühren bei 90°C wird der Ansatz wie in Beispiel 1 aufgearbeitet.

Es wurden 45,6 Teile Diaminodinitril mit einem Reingehalt von 78,7 % und einem Gehalt von 9,9 % an

EP 0 275 519 B1

Mononitril, entsprechend einer Ausbeute von 66 % d.Th. isoliert.

Vergleichsbeispiel 2

In eine Mischung aus 160 Teilen Wasser, 20 Teilen Ethylenglykol und 15 Teilen 50 %iger Natronlauge werden bei 60°C 65,4 Teile 1,4-Diaminoanthrachinon-2-sulfonsäure (Reingehalt: 91,7 %) eingetragen. Mit weiterer Natronlauge wird der pH-Wert auf 7,5 bis 8,0 gestellt. Zu der blauen Lösung gibt man 15,6 Teile m-Nitrobenzolsulfonsaures Natrium, 38 Teile Natriumbicarbonat und 37,2 Teile Natriumcyanid und heizt auf 90°C. Die Reaktion ist nach 24 h laut Dünnschichtchromatogramm noch nicht beendet.

Beim Aufarbeiten gemäß Beispiel 1 isoliert man 45,6 Teile 1,4-Diaminodinitril mit einem Reingehalt von nur 69 %.

Vergleichsbeispiel 3

Man verfährt wie in Vergleichsbeispiel 2, verwendet jedoch anstelle von Ethylenglykol 20 Teile Triethylenglykolmonobutylether. Die Reaktion ist nach 8stündigem Rühren bei 90°C beendet.

Das Reaktionsgemisch wird wie in Beispiel 1 aufgearbeitet.

Ausbeute: 45,3 Teile Diaminodinitril mit einem Reingehalt von 72,8 %; dies entspricht einer Ausbeute von 60 % d.Th.

Vergleichsbeispiele 3 bis 14

Man verfährt wie in Vergleichsbeispiel 2, verwendet jedoch anstelle von Ethylenglykol 20 Teile der in der nachfolgenden Tabelle, Spalte 2, angegebenen Lösungsmittel. Ausbeute und Reingehalt des erhaltenen Dinitrils sowie der Gehalt an Mononitril sind in der Tabelle aufgeführt.

| Vergleichs-beispiel | Lösungsmittel | Diaminodinitril | | Gehalt an Mononitril |
|---|---|---|---|---|
| | | Ausbeute % d.Th. | Reinge-halt % | % |
| 4 | Ethylenglykol-monomethylether | 68,2 | 69 | 4,5 |
| 5 | Diethylenglykol-monomethylether | 67,8 | 70,4 | 3,5 |
| 6 | Ethylenglykol-monobutylether | 68,0 | 71,6 | 4,2 |
| 7 | Diethylenglykol-monobutylether | 65,2 | 75,4 | 3,6 |
| 8 | Ethylenglykol-monophenylether | 67,9 | 74,4 | 3,7 |
| 9 | n-Propanol | 67,6 | 73,2 | 1,7 |
| 10 | i-Butanol | 53,6 | 58,7 | 3,8 |
| 11 | n-Butanol | 52,8 | 69,0 | 4,2 |
| 12 | n-Pentanol | 46,8 | 72,7 | 5,2 |
| 13 | Dipropylenglykol-monomethylether | 69,9 | 75,4 | 0,6 |
| 14 | Dipropylenglykolmono-isopropylether | 57,9 | 75,5 | 4,0 |

5

**Ansprüche**

1. Verfahren zur Herstellung von 1, Diamino-2,3-dicyanoanthrachinon durch Umsetzen von 1, 4-Diaminoanthrachinon-2-sulfonsäure, bzw. von deren Salz, mit Cyanidionen in Wasser im pH-Bereich von 8 bis 11 in Gegenwart von oxidierend wirkenden Mitteln in der Wärme, dadurch gekennzeichnet, daß die Umsetzung in einem Gemisch aus Wasser und 1-Methoxipropanol-2, 2-Methoxipropanol-1, N,H-Dimethylformamid, N,N-Dimethylacetamid, N,N-Dimethylpropionsäureamid, Dimethylsulfoxid, Dioxan, N-Methylpyrrolidon oder Gemischen davon erfolgt, wobei das Verhältnis Wasser zu organischer Flüssigkeit ≧ 3:1 Gew.-Teile ist und wobei das Gemisch auf deu für deu Beginn der Uusefzuug erfoderlideu pH-Bereich vou 8-9 gepuffertwird.

2. Verfahren gemaß Anspruch 1, dadurch gekennzeichnet, daS man als organische Flüssigkeit 1-Methoxipropanol-2, Dimethylformamid, Dimethylacetamid, N-Methylpyrrolidon, Dimethylsulfoxid oder Dioxan verwendet.

3. Verfahren gemäß Anspruch 1 oder 2, dadurch gekennzeichnet, daB das Verhältnis Wasser zu organischer Flüssigkeit 4:1 bis 10:1 Gew.-Teile beträgt.

4. Verfahren gemäß den Ansprüchen 1, 2 oder 3, dadurch gekennzeichnet, daß man - bezogen auf 1 ,4-Diaminoanthrachinon-2-sulfonsäure - die 2,5-bis 5fache Gewichtsmenge an dem Gemisch aus Wasser und organischer Flüssigkeit anwendet.

5. Verfahren gemäß den Ansprüchen 1 bis 4, dadurch gekennzeichnet, daß die Umsetzung in Gegenwart von Natriumhydrogencarbonat als Puffer erfolgt.

6. Verfahren gemäß den Ansprüchen 1 bis 5, dadurch gekennzeichnet, daß das Verhältnis von cyanidabgebender Substanz zu 1,4-Diaminoanthrachinon-2-sulfonsäure 3:1 bis 5:1 Mol beträgt.

7. Verfahren gemäß den Ansprüchen 1 bis 6, dadurch gekennzeichnet, daß die Umsetzung in Gegenwart von m-flitrobenzolsulfonsäure als Oxidationsmittel durchgeführt wird.

**Claims**

1. A process for preparing 1,4-diamino-2,3-dicyano anthraquinone by reacting 1, 4-diaminoanthraquinone-2-sulfonic acid or a salt thereof with cyanide ions in water within the pH range from 8 to 11 in the presence of an oxidizing agent at elevated temperatures, which comprises carrying out the reaction in a mixture of water and l-methoxy-2-propanol, 2-methoxy-l-propanol, N,N-dimethyl-formamide, N, N-dimethylacetamide, N, N-dimethylpropionamide, dimethyl sulfoxide, dioxane, N-methylpyrrolidone or a mixture thereof, the ratio of water: organic liquid being ≧ 3:1 parts by weight and the mixture being buffered to the pH range of 8-9 required for the start of the reaction.

2. A process as claimed in claim 1, wherein the organic liquid used is 1-methoxy-2-propanol, dimethylformamide, dimethylacetamide, N-methylpyrrolidone, dimethyl sulfoxide or dioxane.

3. A process as claimed in claim 1 or 2, wherein the ratio of water: organic liquid ranges from 4:1 to 10:1 parts by weight.

4. A process as claimed in claim 1 or 2 or 3, wherein, based on l,4-diaminoanthraquinone-2-sulfonic acid, from 2.5 to 5 times the weight of a mixture of water and organic liquid is used.

5. A process as claimed in claim 1 or 2 or 3 or 4, wherein the reaction is carried out in the presence of sodium bicarbonate as buffer.

6. A process as claimed in claim 1 or 2 or 3 or 4 or 5, wherein the ratio of cyanide donor substance: 1,4-diaminoanthraquinone-2-sulfonic acid ranges from 3:1 to 5:1 mol.

7. A process as claimed in claim 1 or 2 or 3 or 4 or 5 or 6, wherein the reaction is carried out in the presence of m-nitrobenzenesulfonic acid as oxidizing agent.

**Revendications**

1. Procédé de préparation de 1,4-diamino-2,3-dicyanoanthraquinone par conversion d'acide 2-sulfonique de 1,4-diaminoanthraquinone, par exemple de ses sels, avec des ions cyanures dBns de l'eau dans une plage de pH de 8 á 11 en présence de moyens á action oxydanxe á la chaleur caractérisé en ce que la conversion est réalisée dans un mélange d'eau et de l-méthoxy d'alcool isopropylique, de 2-méthoxy d'alcool propylique, de N,N-diméthylformamide, de N,N-diméthylacétamide, de N,N-diméthylamide d'aclde propionique, d'oxyde sulfonlque de diméthyle, de dioxanne, de N-méthylpyrrolidone ou de mélanges de ceux-ci, dans lequel le rapport entre l'eau et le liquide organique est de 3:1 parties en poids et dans lequel ce mélange esť tamponné dans une plage de pH de 8-9 requise pour le début de la conversion.

2. Procédé suivant la revendication 1, caractérisé en ce qu'on utilise en tant que liquide organique du 1-méthoxy d'alcool isopropylique, du dimèthylformamide, du diméthylacètamide, de la N-méthylyrrcolidone, de l'oxyde sulfonique de diméthyle ou de la dioanne.

3. Procédé suivant la revendication 1 ou 2, caractérisé en ce que le rapport entre l'eau et le liquide organique s'élève à 4:1 à 10:1 parties en poids.

4. Procédé suivant les revendications 1, 2 ou 3, caractérisé en ce qu'on utilise d 2,5 à 5 fois de mélange, en poids, calculé par rapport à l'acide 2-sulfonique de 1,4-diaminoanthraquinone, du mélange eau et liquide organique.

5. Procédé suivant les revendications 1 à 4, caractérisé en ce que la conversion se fait en présence de bicarbonate de sodium en tant que tampon.

6. Procède suivant les revendications 1 a 5, caractérisé en ce que le rapport entre la substance libérant du cyanure et l'acide 2-sulfonique de 1,4-diaminoanthraquinone s'élève à 3:1 à 5:1.

7. Procédé suivant les revendications 1 à 6, caractérisé en ce que la conversion est réalisée en présence d'acide sulfonique de m-nitrobenzène en tant que moyen d'oxydation.